# EUROPEAN PATENT APPLICATION

(11) **EP 4 292 608 A1**
(43) Date of publication of application: **20.12.2023**
(21) Application number: 22178787.2
(22) Date of filing: 14.06.2022
(51) Int. Cl.: A61K 39/395, A61K 35/17, A61P 35/00

(54) **DOSAGE REGIMEN COMPRISING ADMINISTRATION OF TUMOR INFILTRATING LYMPHOCYTES AND A CHECKPOINT INHIBITOR**

(71) Applicant: CuraCell Germany GmbH, 69117 Heidelberg (DE)
(72) Inventor: WINQVIST, Ola, 756 53 Uppsala (SE)
(74) Representative: Aera A/S

(57) **Abstract**

A dosage regimen for use in the treatment of cancer comprising administration of *in vitro* expanded tumor infiltrating lymphocytes, TILs, to a mammal suffering from cancer at day 0(TIL), followed by administration of a checkpoint inhibitor at day 0(CI), wherein day 0(CI) is in a range of from 1 months to 5 months later than day 0(TIL) such as from 6 weeks to 4 months, from 8 weeks to 3 months or about 2 months later than day 0(TIL).

## Description

### FIELD OF THE INVENTION

The present invention relates to the use of a combination of tumor infiltrating lymphocytes and one or more check-point inhibitors for the use in the treatment of cancer such as prostate cancer.

### BACKGROUND OF THE INVENTION

Cancer cells are characterized by a myriad of genetic mutations and epigenetic alterations that give rise to a large variety of cancer-specific antigens. These antigens are detected by T cells, which utilize the antigens to distinguish precancerous and/or cancerous cells from their normal counterparts and elicit a cancer-specific immune response. The amplitude and quality of the T-cell-mediated immune response is normally regulated by immune checkpoints, which can be defined as stimulatory and inhibitory molecules and/or molecular pathways acting to increase or decrease, respectively, the magnitude of a response. Under normal physiological conditions, immune checkpoints are crucial for the prevention of autoimmunity and protection from tissue damage resulting from pathogenic infections. However, cancer cells may utilize dysregulation of immune checkpoint proteins as a way to obtain immune resistance.

One approach to trigger T cell-mediated antitumor immune responses has been termed "checkpoint blockade", referring to the blockade or inhibition of immune-inhibitory checkpoints that are utilized by cancer cells. Since many immune checkpoints are initiated by ligand-receptor interactions, these checkpoints may be blocked by antibodies or modulated by recombinant forms of the ligands and/or receptors in question.

Advanced prostate cancer is associated with poor prognosis and remains incurable. Apart from Sipuleucel-T, an FDA-approved therapeutic cancer vaccine, effective immunotherapeutic approaches for prostate cancer still remain challenging.

Immune-checkpoint inhibitors (anti-PD-1/anti-PD-L1/anti-CTLA4) that are effective in different malignancies show only limited activity in prostate cancer, partially due to the relatively low mutational burden in this disease. Response rates, e.g., with pembrolizumab, are reported with 5 and 3 % only.

Therefore, new immunotherapeutic strategies are urgently needed.

Adoptive therapy with tumor infiltrating T lymphocytes (TILs) has shown remarkable results in the treatment of individual patients with different types of cancer in the last decades, with the most recent successful clinical case reported in 2018. The recognition of multiple individual neo-antigens as well as shared tumor-associated antigens (TAAs) linked to enhanced tissue homing capacity and strong immune effector functions jointly contribute to the clinical efficacy of TIL in patients with solid tumors. TIL therapy has shown clinical benefit for patients with chemotherapy-refractory cancer, such as metastatic melanoma, cholangio-cellular carcinoma, renal cell carcinoma, colorectal, cervical-, cervical-, breast- and ovarian cancer. Currently only few clinical studies investigating adoptive transfer of T cells for metastatic hormone-refractory prostate cancer (mHRPC) are listed on www.clinicaltrials.gov.

### SUMMARY

The present invention provides a novel dosage regimen for the treatment of cancer. The cancer is typically a cancer that is not effectively responsive to sole treatment with a checkpoint inhibitor and/or it is a cancer with relatively low mutational burden. An example of such a cancer is prostate cancer.

In its broadest aspect the invention relates to a a dosage regimen for use in the treatment of cancer comprising administration *of in vitro* expanded tumor infiltrating lymphocytes, TILs at day 0(TIL) to a mammal suffering from cancer, followed by administration of a checkpoint inhibitor at day 0(CI), wherein day 0(CI) is in a range of from 1 months to 5 months later than day 0(TIL) such as from 6 weeks to 4 months, from 8 weeks to 3 months or about 2 months later than day 0(TIL).

The treatment may be preceded with one or more administrations of TILs and may be followed by one or more administrations of TIL and/or one or more administrations of a checkpoint inhibitor.

Specifically, the present invention relates to a dosage regimen for use in the treatment of cancer comprising administration of *in vitro* expanded tumor infiltrating lymphocytes, TILs, (TIL-2) at day 0(TIL-2) to a mammal suffering from cancer, followed by administration of a checkpoint inhibitor at day 0(CI), wherein day 0(CI) is in a range of from 1 months to 5 months later than day 0(TIL-2) such as from 6 weeks to 4 months, from 8 weeks to 3 months or about 2 months later than day 0(TIL-2).

In the present context, the term "tumor infiltrating lymphocytes" or "TILs" or "TIL" is intended to mean T lymphocytes carrying a T cell receptor (TCR) specific for and recognizing a tumor antigen. The term is similar to the term "tumor-reactive T lymphocytes. The term also covers T lymphocytes carrying a TCR specific for and recognizing metastasis antigens, i.e. the terms "tumor infiltrating lymphocytes"/"TILs"/"TIL" encompass "metastasis infiltrating lymphocytes."

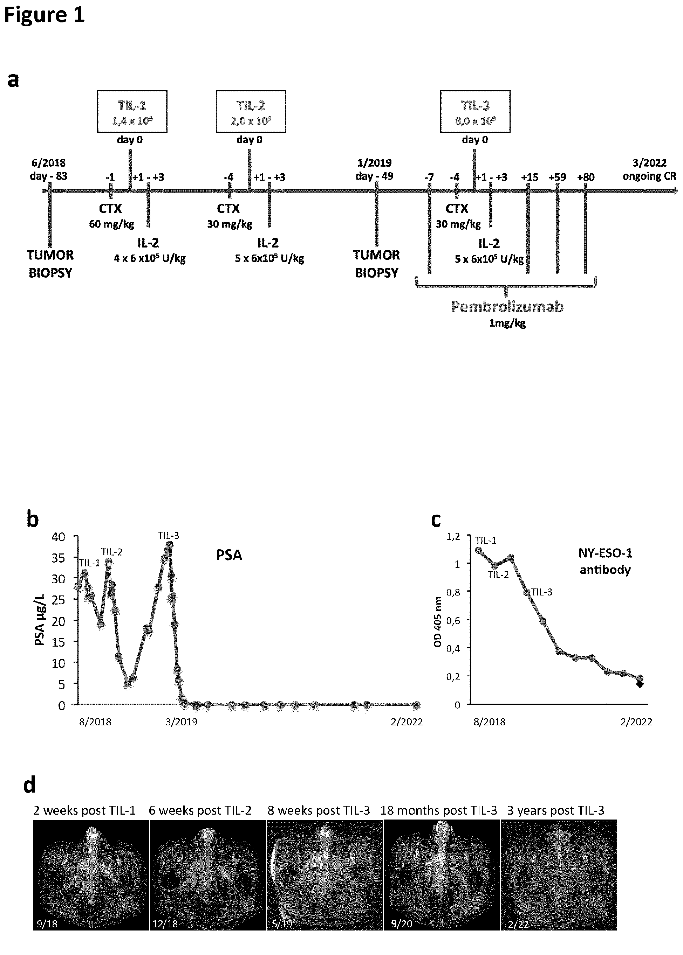

In the dosage regime it is important that the TILs are administered before the checkpoint inhibitor. It is contemplated that the immunotherapeutic effect initiated by TILs makes the cancer susceptible for treatment with a checkpoint inhibitor. As seen from the examples herein a remarkable effect is achieved in a patient suffering from prostate cancer and subjected to the dosage regime described herein (see Fig. 1b and 1c).

The invention also relates to a kit for use in the treatment of cancer, the kit comprising
i) in vitro expanded tumor infiltrating lymphocytes such as TIL-1, TIL-2 and/or TIL-3, and
ii) one or more checkpoint inhibitor,
wherein the kit comprises two or more separate components, wherein a first component comprises one or more compositions comprising *in vitro* expanded tumor infiltrated lymphocytes and a second component comprises one or more compositions comprising a checkpoint inhibitor.

### DETAILED DESCRIPTION OF THE INVENTION

The present invention provides a novel dosage regimen for the treatment of cancer. The dosage regimen comprises sequential administration of tumor infiltrating lymphocytes (TILs) preceded at certain occasions by administration of a checkpoint inhibitor and, at certain occasions, followed by administration of a checkpoint inhibitor. The tumor infiltrating lymphocytes are obtained by *in vitro* culturing lymphocytes obtained from a biopsy or a body sample containing TILs of a patient suffering from cancer and administering the thus obtained tumor infiltrating lymphocytes (TILs) to said patient. The *in vitro* expansion of tumor infiltrating lymphocytes is performed in a culture medium containing one of more of IL-2, IL-15 and IL-21.

The dosage regimen in its simplest form comprises the following steps:
i) Administration *of in vitro* expanded tumor infiltrating lymphocytes, TILs, at day 0(TIL) to a mammal suffering from cancer, followed by
ii) administration of a checkpoint inhibitor at day 0(CI), wherein day 0(CI) is in a range of from 1 months to 5 months later than day 0(TIL) such as from 6 weeks to 4 months, from 8 weeks to 3 months or about 2 months later than day 0(TIL).

As mentioned above, it is important that the TILs are administered to the subject suffering from cancer before treatment with a checkpoint inhibitor is initiated. Many cancer types have a relatively low mutation frequency such as a somatic mutation frequency(/Mb) lower than 10 (Lawrence, M.S., Nature 2013, 499, 214-8). Such cancers include rhabdoid tumor, Ewing sarcoma, thyroid cancer, AML, medulloblastoma, glioblastoma, neuroblastoma, carcinoid tumor, prostate cancer, breast cancer and pancreas cancer. The inventors have observed that such cancer types typically also do not effectively respond to treatment with a checkpoint inhibitor, but - as described herein - an effective treatment regimen can be obtained by a dosage regimen involving a pre-treatment of the cancer with TILs followed by treatment with a checkpoint inhibitor. The treatment may be repeated as many times as needed and the checkpoint inhibitor may be administered as many times as needed even after the last treatment with TILs.

As an example of a dosage regime of the invention is given a dosage regimen comprising three administrations of TILs. A person skilled in the art will know that more administrations of TILs may precede the administration of TIL-1, and more administrations of TILs may follow administration of TIL-3.

A non-limiting example of a dosage regimen is given in the following. The time periods between the administration of TILs and checkpoint inhibitors and between administration of dosages of TILs are general and applies irrespectively of how many doses of TILs or checkpoint inhibitors that are given:
Administration *of in vitro* expanded tumor infiltrating lymphocytes, TILs, (TIL-2) at day 0(TIL-2) to a mammal suffering from cancer, followed by
ii) administration of a checkpoint inhibitor at day 0(CI), wherein day 0(CI) is in a range of from 1 months to 5 months later than day 0(TIL-2) such as from 6 weeks to 4 months, from 8 weeks to 3 months or about 2 months later than day 0(TIL-2).

The dosage regimen may further comprise administration *of in vitro* expanded tumor infiltrating lymphocytes, TILs, (TIL-3) to said mammal at day 0(TIL-3), wherein day 0(TIL-3) is in a range of from 1 to 14 days after day 0(CI) such as from 2 to 12 days, from 5 to 12 days or 7 days after day 0(CI). Thus, after the administration of a checkpoint inhibitor a further treatment with TILs may be necessary. Even before administration of TIL-2 administration of TILs may take place. Thus, the dosage regimen may further comprise administration of i*n vitro* expanded tumor infiltrating lymphocytes, TILs, (TIL-1) to said mammal at day 0(TIL-1), wherein day 0(TIL-1) is in a range of from 1 to 4 months before day 0(TIL-2) such as from 6 weeks to 4 months, from 8 weeks to 3 months or about 2 months before day 0(TIL-2).

The administration frequency and number of cells (TILs) administered depend on how effective the treatment is. The treatment may be followed by monitoring markers specific for the cancer type, such as, e.g., PSA for prostate cancer. In the case of prostate cancer, an increase in PSA is indicative of progression of the cancer and indicative of that a further treatment with TILs and/or a checkpoint inhibitor should be given. In general, TILs may be administered from 1 to 10 times such as from 2 to 8 times, from 2 to 6 times, from 2 to 5 times, from 3 to 4 times such as 3 times. The time period between the administrations of TILs is from 1 to 6 months such as from 6 weeks to 6 months, from 8 weeks to 5 months, from 8 weeks to 4 months, from 8 weeks to 3 months or about 2 months.

Typically, the day for administering a second (third, fourth etc.) dose of TILs may be determined by monitoring the plasma level of a marker specific for said cancer, a tumor-associated antigen specific for said cancer or a neoantigen specific for said cancer, wherein the time period for monitoring starts after administration of first portion of TILs. When the level is changed by a factor 4 or more, the second (third, fourth etc.) dose of TILs is administered 1 day to 1 months, such as 1 day to 3 weeks, 1 day to 2 weeks or 1 day to 7 days after such a change has been observed. The change may be an increase or a decrease in the level of the marker monitored dependent on the type of cancer. In the case of prostate cancer, the level of PSA is monitored and an increase in the PSA level is indicative in progression of the disease and therefore the above-mentioned change is an increase in the PSA level. A generally applicable marker is NY-ESO-1 and here, the change is also an increase in the NY-ESO-1 antibody level.

The number of TILs administered is typically in a range of from 1 × 10⁸ to 1 × 10¹¹, such as from 1 × 10⁸ to 1 × 10¹⁰ such as from 1 × 10⁹ to 1 × 10¹⁰.

In the dosage regimen the number of TILs administered may be increased during the treatment period, e.g., the number may increase from TIL-1 to TIL-2 and to TIL-3.

Apart from administration of TILs, a chemotherapeutic agent may be administered. Such a chemotherapeutic agent is typically administered from 7 to 1 day before administration of TILs (such as, e.g., TIL-1, TIL-2 and/or TIL-3). Examples on chemotherapeutic agents suitable for use include cyclophosphamide (CTX) and fludarabine. Such chemotherapeutic agents are contemplated to have a Treg downregulating effect that can downregulate the cytotoxic potential of TILs.

In aspects, said chemotherapeutic agent is an agent normally used in the treatment of said cancer, but where the dose is lowered compared to the dose normally use in the treatment of said cancer.

The dosage regimen may also include administration of e.g., IL-2. IL-2 is typically administered from 1 to 5 days after administration of TILs such as from 1 to 3 days after administration of TILs. The dose of IL-2 is typically in a range from 6 × 10⁵ IU/kg body weight to 5 × 6 × 10⁶ IU/kg body weight such as from 2 × 6 × 10⁵ IU/kg body weight to 8 × 6 × 10⁵ IU/kg body weight, from 3 × 6 × 10⁵ IU/kg body weight to 7 × 6 × 10⁵ IU/kg body weight, from 4 × 6 × 10⁵ IU/kg body weight to 6 × 6 × 10⁵ IU/kg body weight or from 4 × 6 × 10⁵ IU/kg body weight to 5 × 6 × 10⁵ IU/kg body weight.

In aspects of the invention, the subject suffering from cancer has not been subject to chemotherapy in a time period of at least 6 months before start of the dose regimen of the present invention.

As demonstrated in the Examples, repeated administration of TILs as opposed to single infusions was found to improve the clinical outcome by prolonging the phase of TILs-tumor interactions in vivo and by reducing treatment-related toxicities.

As shown in Figure 1a, the novel dosage regime may comprise infusion of TILs at one or more times. In general infusion of TILs three times had led to remarkable results. Thus, a method of the invention may include TIL-1, TIL-2 and TIL-3, where TIL-1 is the first portion of TILs to be infused, TIL-2 the second portion and TIL-3 the third portion. Dependent on the development (progression, regression or relapse), a fewer number of infusions may be required, or a larger number of infusions may be required. In the case exemplified in the examples, the development of the disease is followed using the presence in plasma or serum of a marker for the cancer disease (PSA for prostate cancer) and/or the presence of a specific tumor-associated antigen (such as, e.g., NY-ESO-1) or a neoantigen.

Based on the relevant parameter for the specific cancer type, the development of the disease can be followed and a person skilled in the art will be able to determine with to stop the treatment or when to repeat the treatment.

The novel dosage regimen is based on the observation that initial administration of TILs had some effect, but the disease relapses after a while (see Figure 1b), i.e only a transient efficacy could be obtained. The transient response might be explained by either a too low number of TILs, impaired persistence of transferred T-cells or due to an inadequate migration of the transferred T-cells into the tumor microenvironment). To improve the outcome and to achieve a durable response, further infusions with a higher number of cells (TIL-2 and/orTIL-3) can be used. Moreover, it seems that a dosage regime involving a first administration of TILs (optionally repeated one or more time) followed by administration of a check-point inhibitor is effective. It is contemplated that initiating the treatment with tumor infiltrating lymphocytes having homing markers (e.g., NY-ESO-1) for the cancer/tumor is beneficial before treatment with a check-point inhibitor is initiated. As described herein, the use of check-point inhibitors in the treatment of e.g., prostate cancer has not been successful, but as reported herein, a treatment involving an initial treatment with TILs followed by administration of a check-point inhibitor is successful and a patient has been free of symptoms now for 3 years.

The TILs used according to the invention may be autologous or allogenic.

### Checkpoint inhibitors

As mentioned above, in the novel dosage regimen, TILs will be used in combination with a checkpoint inhibitor.

Checkpoint inhibitor therapy is a form of cancer immunotherapy. Checkpoint inhibitors target immune checkpoints, key regulators of the immune system, which when stimulated can dampen the immune response to an immunologic stimulus. Checkpoint therapy can block inhibitory checkpoints thereby restoring immune system function. The first anti-cancer drug targeting an immune checkpoint was ipilimumab, a CTLA4 blocker.

Currently approved checkpoints inhibitors target the molecule CTLA4, PD1 and PD-L1. PD-1 is the transmembrane programmed cell death 1 protein (also called PDCD1 or CD279), which interacts with PD-L1, PD-1 ligand 1). PD-L1 on the cell surface binds to PD-1 on an immune cell surface, which inhibits immune cell activity. Among PD-L1 functions is a key regulatory role on T cell activities. It appears that cancer-mediated upregulation of PD-L1 on the cell surface may inhibit T cells that might otherwise attack. Antibodies that bind to either PD-1 or PD-L1 and therefor block the interaction may allow the T cells to attack the tumor.

Several immune checkpoints, either alone or in combination, are relevant in terms of enhancing T cell-mediated antitumor immune responses. These include, but are not limited to, cytotoxic T-lymphocyte associated antigen 4 (CTLA4, also known as CD152), programmed cell death protein 1 (PD-1, also known as CD279), PD-1 ligand 1 (PD-L1, also known as B7-H1 and CD274), PD-1 ligand 2 (PD-L2, also known as B7-DC and CD-273), T-cell membrane protein 3 (TIM3, also known as HAVcr2), adenosine A2a receptor (A2aR), lymphocyte activation gene 3 (LAG3, also known as CD 223), and B7-H3 (also known as CD276), B7-H4 (also known as B7-S1, B7X, and VCTN1), 2B4 (also known as CD244), and B and T lymphocyte attenuator (BTLA, also known as CD272).

Of particular interest are immune checkpoint inhibitors selected from inhibitors of PD-1 (such as nivolumab, pembrolizumab or cemiplimab), PD-L1 (such as atezolizumab, avelumab or durvalumab) and CTLA-4 (such as ipilimumab).

Moreover, other examples of relevant immune checkpoints can be found in the scientific and patent literature and are also within the scope of the present invention.

Although immune checkpoint inhibition is useful for enhancing T cell-mediated anti- tumor immunity, it is contemplated by the present inventors that combining immune checkpoint inhibition with one or more complementary mechanisms to further enhance T cell activation will provide even better antitumor effects.

### In vitro expansion of TILs

In general, various different methods can be used to expand TILs obtained from a subject suffering from cancer.

One method involves the use of three different cytokines, IL-2, IL-15 and IL-21. A method may also involve the use of IL-15 and IL-21, i.e without the use of IL-2, or a method may involve the use of IL-2. When IL-2, IL-15 and IL-21 are used, the method involves two phases, where the first phase i) is to obtain a culture comprising a substantially high ratio of TIL, notably CD8+ T-lymphocytes. In this phase the TIL are brought to survive and divide. The starting material for the first phase may be a mixture of lymphocytes and antigen presenting cells provided from biopsy or a body sample from a subject suffering from cancer.

The T-lymphocytes to be expanded in culture can be obtained from the subject to be treated, i.e., the resulting TILs for administration may be autologous. However, the T-lymphocytes can also be obtained from a source other than the subject to be treated such as, e.g., another subject suffering from a cancer. In such a case the recipient and the expanded TILs are preferably immunologically compatible, or the recipient is otherwise made immunotolerant of the expanded TILs. In one method, the first phase is initiated by adding a cytokine cocktail containing IL-2, IL-15 and IL-21 to stimulate the TIL via the cytokine receptors to promote cell division and to prevent cell death. In another method only IL-2 is used to stimulate TILs via the IL2-receptor. The use of either a cytokine cocktail or IL-2 alone aims to promote specific activation and growth of CD4+ helper lymphocytes and CD8+ lymphocytes. Such a specific activation against a certain tumor antigen enables the TILs to have therapeutic effect when administered to a cancer patient with the same tumor type as the TILs are activated against.

The second phase ii) aims at clonal expression of the TILs from phase. In the second phase, the culture medium containing either the cytokine cocktail or IL-2 is supplemented with human serum, feeder cells, an anti-human CD3 or CD28 antibody.

The expansion of the TILs in phase i) and ii) will most often take place in a suitable culture medium. Preferably a serum-free medium or autologous serum is used in order to avoid the risk of transmitting diseases to the patient. Examples of suitable standard media include AIMV medium, RPMI 1640, DMEM and MEM. However, other media may also be used, comprising a suitable blend of amino acids, steroids, vitamins, growth factors, other cytokines and minerals.

In the example herein, TIL-1 is obtained by expanding TILs using IL-2, IL-15 and IL-21. The combination of the three cytokines lead to the expansion of a high percentage of CD8+ cytotoxic T-cells (78-95%) and a relatively low number of CD4+ T-cells (11-1,7%), with the majority of TIL being effector memory T cells (CD45RA- CCR7+). In addition, this culture method did not promote outgrowth of Tregs.

Thus, characteristics of the TILs cell population are:
i) the content of CD8+ cytotoxic T-cells is in a range of from 70% to 98% such as in a range of from 75% to 97% or in a range of from 78% to 95% based on the total number of T-cells.
ii) the content of CD4+ T-cell is in a range of from 0.5% to 20% such as from 1% to 15%, from 1.5% to 12% based on the total number of T-cells.
iii) the content of effector memory T-cells that are CD8 positive (CD45RA- CCR7+) is in a range of from 45 to 80% such as from 45 to 78%.

IL-2, IL-15 and IL-21 are members of the cytokine family each of which has a four alpha helix bundle. IL-2 has key roles in key functions of the immune system, tolerance and immunity, primarily via its direct effects on T-cells. IL-2 induces T- cell proliferation and differentiation into effector and memory T-cells.

IL-15 is a cytokine that is structurally similar to IL-2. Like IL-2, IL-15 binds to and signals through a complex composed of the IL-2/IL-15 receptor beta chain. IL-15 induces a T-cell activation and proliferation in particular of CD8+ T-cells (30) and also provides survival signals to maintain memory cells in the absence of antigens, favored CD8+ T-cells and activates monocytes. IL-15 appears to drive rather 20 proliferation of immune effector T-cells, along with the protection from inhibition of tumor-associated immunosuppression.

IL-21 is a cytokine that has potent regulatory effects on cells of the immune system, including natural killer (NK) cells and cytotoxic T-cells. IL-21 enriches central memory type T-cells with a CD28+ CD127hi CD45RO+ phenotype and enhances the cytotoxity of cytotoxic T-cells. IL-21 may keep T-cells in their early phase of differentiation and maturation.

As used herein, "interleukin 2" or "IL-2" refers to human IL-2 as defined by SEQ ID NO: 9 and functional equivalents thereof. Functional equivalents of IL-2 include relevant substructures or fusion proteins of IL-2 that remain the functions of IL-2. Accordingly, the definition IL-2 comprises any protein with a sequence identity to SEQ ID NO: 9 of at least 80 %, preferably at least 90 %, more preferably at least 95 %, most preferably at least 98 %. Recombinant human IL-2 produced in E coli as a single, non-glycosylated polypeptide chain with 134 amino acids and having a molecular mass of 15 kDa is commercially available in lyophilized form from Prospec as CYT-209.

As used herein, "interleukin 15" or "IL-15" refer to human IL-15 and functional equivalents thereof. Functional equivalents of IL-15 include relevant substructures or fusion proteins of IL-15 that remain the functions of IL-15. Accordingly, the definition IL-15 comprises any protein with a sequence identity to SEQ ID NO: 10 of at least 80 %, preferably at least 90 %, more preferably at least 95 %, most preferably at least 98 %. Recombinant human IL-15 produced in E. coli as a single, non-glycosylated polypeptide chain with 114 amino acids (and an N- terminal Methionine) and having a molecular mass of 12.8 kDa is commercially available in lyophilized form from Prospec as CYT-230.

As used herein, "interleukin 21" or "IL-21" refer to human IL-21 and functional equivalents thereof. Functional equivalents of IL-21 included relevant substructures or fusion proteins of IL-21 that remain the functions of IL-21. Accordingly, the definition IL-21 comprises any protein with a sequence identity to SEQ ID NO: 11 of at least 80 %, preferably at least 90 %, more preferably at least 95 %, most preferably at least 98 %. Recombinant human IL-21 produced in E. coli as a single, non-glycosylated polypeptide chain with 132 amino acids and having a molecular mass of 15 kDa is commercially available in lyophilized form from Prospec as CYT-408.

A "peptide" as used herein may be composed of any number of amino acids of any type, preferably naturally occurring amino acids, which, preferably, are linked by peptide bonds. In particular, a peptide comprises at least 3 amino acids, preferably at least 5, at least 7, at least 9, at least 12, or at least 15 amino acids. However, preferably, a peptide for use according to the invention does not exceed a length of 500 amino acids, more preferably it does not exceed a length of 300 amino acids; even more preferably it is not longer than 250 amino acids.

Apart from the cytokines, the culture medium in which the TILs are expanded may contain various factors such as growth factors such as those normally used in expansion of lymphocytes.

The TILs expanded by the above method/methods all show reactivity against tumor-associated antigens. In particular, as seen from the example therein, TILs expanded from cells obtained from biopsy from a subject suffering from prostate cancer showed reactivity against NY-ESO-1. As explained herein, the peptide panel used for the identification of TIL specificities did not represent the full range of TAA and neo-antigens expressed in the tumor. T cells reactive against other target antigens, e.g. MAGE, expressed on the autologous tumor, may contribute to the clinical outcome.

### Tumor-associated antigens (TAAs)

Tumor-associated antigens are antigenic substances produced in tumor cells and they trigger an immune response in the host. TAA are useful tumor markers in identifying tumor cells with diagnostic tests and are potential candidates for use in cancer therapy.

In the present context, TAAs are antigens that are presented by MHCI or MHCII molecules or non-classical MHC molecules on the surface of the tumor cells. As used herein TAA includes tumor-specific antigens, which are found only on the surface of tumor cells, but not on the surface of normal cells.

Tumor-associated antigens are antigens that are involved in a cancer disease.

In the present context, an antigen is any structural substance, which serves as a target for the receptors of an adaptive immune response, TCR, or antibody, respectively. In particular, antigens are proteins, polysaccharides, lipids, or substructures thereof such as peptides. Lipids and nucleic acids may be antigenic when combined with proteins or polysaccharides.

TAAs for use in the present invention include, e.g., cancer-testis antigens such as, e.g., MAGE-A1, MAGE-A3, MAGE-A4, NY-ESO-1-, PRAME, CT83, SSX3, NY-ESO-1,

Cancer-testis antigens are a group of proteins united by their importance in development and in cancer immunotherapy. In general, expression of these proteins is restricted to male germ cells in the adult animal. However, in cancer these developmental antigens are often re-expressed and can serve as a locus for immune activation.

Other TAAs for use according to the invention include survivin, GPI, EGRvrlll, tyrosinase tumor antigen, tyrosinase related protein (TRP)-I, TRP-2, VEGFR-2, a member of the MAGE family of proteins (can be found in melanoma), telomerase, p53, HER2/neu, mesothelin, carcinoembryonic, VEGF, CAMPATH 1-antigen, CD22, CA-125 (can be found in ovarian cancer), MUC-1 (can be found in breast cancer), Epithelial tumor antigen (can be found in breast cancer) Mucin-1, Alpha-1-fetoprotein (can be found in germ cell tumors or hepatocellular carcinoma), PSMA, RAS, or substructures or fragments thereof. Other examples are peptides derived from individual tumor mutational proteins PRPF8, TRPS1 and the androgen receptor splice variant-12.

A particular suitable TAA for use according to the invention depends on the type of cancer to be treated.

The fragment of the TAA is in particular a peptide. Such peptide can be for example a peptide comprising at least eight continuous amino acids of an amino acid sequence that is at least 80 % identical to the amino acid sequence of SEQ ID NO: 5, SEQ ID NO: 6, and SEQ ID NO: 7.

SEQ ID NO: 5 is the amino acid sequence of the known tumor associated antigen NY-ESO-1. SEQ ID NO: 6 is the amino acid sequence of the known tumor associated antigen survivin. SEQ ID NO: 7 is the amino acid sequence of the known tumor associated antigen mesothelin.

| SEQ ID NO: | Description | Sequence |
|---|---|---|
| 1 | Primer RFPL1_se | CCACATCAAGGAACTGGA |
| 2 | Primer RFPL1_rev | CTGGTCTGGTCGTAGGAA |
| 3 | RFPL1_wt (HEX) | CCTCACCTTGGAACTTC |
| 4 | RFPL1_mut (FAM) | CCTCACATTGGAACTTC |
| 5 | AA sequence of NY-ESO-1 - Gene GTAG1A, Length:1 80 Amino Acids, Mass (Da):17,992 | MQAEGRGTGG STGDADGPGG PGIPDGPGGN AGGPGEAGAT GGRGPRGAGA ARASGPGGGA PRGPHGGAAS GLNGCCRCGA RGPESRLLEF YLAMPFATPMEAELARRSLA QDAPPLPVPG VLLKEFTVSG NILTIRLTAA DHRQLQLSIS SCLQQLSLLM WITQCFLPVF LAQPPSGQRR |
| 6 | AA sequence of survivin Length:142 Mass (Da):16,389 | MGAPTLPPAW QPFLKDHRIS TFKNWPFLEG CACTPERMAE AGFIHCPTEN EPDLAQCFFC FKELEGWEPD DDPIEEHKKH SSGCAFLSVK KQFEELTLGE FLKLDRERAK NKIAKETNNK KKEFEETAKK VRRAIEQLAA MD |
| 7 | AA sequence of mesothelin Length:332 Mass (Da):36,729 | MALPTARPLL GSCGTPALGS LLFLLFSLGW VQPSRTLAGE TGQEAAPLDG VLANPPNISS LSPRQLLGFP CAEVSGLSTE RVRELAVALA QKNVKLSTEQ LRCLAHRLSE PPEDLDALPL DLLLFLNPDA FSGPQACTRF FSRITKANVD LLPRGAPERQ RLLPAALACW GVRGSLLSEA DVRALGGLAC DLPGRFVAES AEVLLPRLGI VAAWRQRSSR DPSWRQPERT ILRPRFRREV EKTACPSGKK AREIDESLIF YKKWELEACV DAALLATQMD RVNAIPFTYE QLDVLKHKLD ELYPQGYPES VIQHLGYLFL KMSPEDIRKW NV |
| 8 | RING finger protein RFP1 Length:392 Mass (Da):43,118 | MVWVKHQIQQ KDGYLSYSPP LLSSSSSSSS SSSSSSPPYP LGATFHKESS LPSSTPPPPP SGTRISPAVL FIIVVLAVLF FISGLLHLLI RFLIKHPSSA SAQSNRHQEL STSDALQRQL QQLFHLHDSG LDQAFIDALP VFQYKEIVGL KEPFDCAVCL CEFSEKDKLR LLPMCSHAFH ISCIDTWLLS NSTCPLCRGT LLTQGFSIEN PIFDFDDLRE DEGCPCNGEN GFNTRQKTVV VEESVEKEKG VFPVRLGKFK KLSVKGGGES EVGETSSSNL DARRCYSMGS YQYVVGNSDL RVTLNHDEIG KGQVTRLTKG MELDDGKVSV EEGDVEAKKI SSVSKGDSFS VSKIWLWPKK GKLPTSLDGQ FGRPMPSYLN TGFPRMREPE GA |
| 9 | IL-2 | MYRMQLLSCIALSLALVTNSAPTSSSTKKTQLQLEHLLL DLQMILNGINNYKNPKLTRMLTFKFYMPKKATELKHLQC LEEELKPLEEVLNLAQSKNFHLRPRDLISNINVIVLELKG SETTFMCEYADETATIVEFLNRWITFCQSIISTLT |
| 10 | IL-15 | MRISKPHLRSISIQCYLCLLLNSHFLTEAGIHVFILGCFSA GLPKTEANWVNVISDLKKIEDLIQSMHIDATLYTESDVHP SCKVTAMKCFLLELQVISLESGDASIHDTVENLIILANNSL SSNGNVTESGCKECEELEEKNIKEFLQSFVHIVQMFINT S |
| 11 | IL-21 | MERIVICLMVIFLGTLVHKSSSQGQDRHMIRMRQLIDIVD QLKNYVNDLVPEFLPAPEDV ETNCEWSAFSCFQKAQLKSANTGNNERIINVSIKKLKRK PPSTNAGRRQKHRLTCPSCDS YEKKPPKEFLERFKSLLQKMIHQHLSSRTHGSEDS |
| 12 | PRPF8_Y471C | KKRCLFRSF |
| 13 | SGIP1_G780C | CPSKPSPLV |
| 14 | STAMBVP_R170Q | MIQNQELEK |
| 15 | TRPS1_H590Y | YLGEITYPF |
| 16 | NID2_P971A | CDEQGNFLALQCHGS |
| 17 | ZNF512_A437S | FVSGKYKCL |
| 18 | GFM1_P534fs | NQVVQASMEK |
| 19 | DHCR7 _I92fs | YLVGHLPGA |
| 20 | CNN1_A128D | VQSTLLALDSMAKTK |
| 21 | AR_Var12 | VKWAKALPDCERAAS |

NY-ESO-1 is a highly immunogenic cancer-testis antigen, inducing simultaneous cellular and humoral immune responses in a high percentage of patients with advanced NY-ESO-1 expressing tumors, and has therefore been considered promising as an effective target for cancer immunotherapy. Serum antibodies against NY-ESO-1 have been found to increase with disease progression and to decrease with disease regression, rendering the antibody a useful biomarker to monitor disease activity and treatment response. Several studies have also indicated that patients with pre-existing NY-ESO-1 antibodies have an increased clinical benefit from immune checkpoint blockade. Adoptive transfer of NY-ESO-1 specific T cells has improved clinical response rates in melanoma, synovial cell sarcoma and multiple myeloma patients.

Herein is reported a patient with advanced prostate cancer who experienced complete and durable tumor remission after treatment with *in vitro* expanded tumor-infiltrating lymphocytes (TILs) that recognized NY-ESO-1 and multiple mutational tumor antigens, potentially relevant for anti-tumor immunity.

### Other aspects of the invention

The invention also provides a dosage regimen for use according to any one of the preceding claims, wherein said TILs has been obtained *in vitro* by the steps of
i) providing a tumor or tumor metastasis sample obtained from said mammal;
ii) isolating TILs from the sample;
iii) culturing said TIL in a medium containing IL-2, IL-15 and/or IL-21,
iv) adding CD3 antibody and feeder cells to the culture medium.

Also disclosed is a kit for use for the treatment of cancer, the kit comprising
i) in vitro expanded tumor infiltrating lymphocytes, TILs, and
ii) one or more checkpoint inhibitor,
wherein the kit comprises two or more separate components, wherein a first component comprises one or more compositions comprising *in vitro* expanded tumor infiltrated lymphocytes and a second component comprises one or more compositions comprising a checkpoint inhibitor.

The kit may further comprise a third component comprising a composition comprising a chemotherapeutic agent.

Said first component may comprise one composition comprising TIL-1, TIL-2 or TIL-3 or it may comprise two compositions, a first composition comprising TIL-1, and a second composition comprising TIL-2.

Said first component may comprise two compositions, a second composition comprising TIL-2, and a third composition comprising TIL-3 or it may comprise three compositions, a first composition comprising TIL-1, a second composition comprising TIL-2 and a third composition comprising TIL-3.

The kit can also contain instructions for use.

### Discussion of the results of the examples

Cell-based immunotherapy of cancer represents a viable method for treating patients with advanced malignancies, leading to durable clinical responses. Herein is reported the clinical and immunological efficacy of *in vitro* expanded TIL in a patient with metastatic prostate cancer. TIL administered three times at relatively low cell numbers led to complete tumor regression lasting now for 3 years. In contrast to other groups who are engaged in TIL-therapy, we followed different strategies for TIL expansion, mode and number of application and conditioning regime, which may have contributed to this extraordinary result.

First, we used a modified culture method for in vitro isolation and expansion of TIL, utilizing of IL-2, IL-15 and IL-21 containing medium and a closed perfusion bioreactor system. We suppose that theses cytokines in combination with the culturing procedure led to the expansion of a high percentage of CD8+ cytotoxic T-cells (78-96%) and a relatively low number of CD4+ T-cells (11-1,7%), with the majority of TIL being effector memory T-cells (CD45RA- CCR7+). In addition, this culture method did not promote outgrowth of Tregs (<0,13%).

Second, a modified preparatory chemotherapy regimen that differs from that employed in TIL studies reported by the US National Cancer Institute, using a single low-dose infusion of cyclophosphamide aiming to reduce regulatory T cells without a higher impact on general lymphodepletion. The single low-dose was given one day (TIL-1) and four days (TIL-2 and TIL-3) proceeding TIL infusion, This helped to significantly reduce treatment-related toxicity in comparison with the widely used high dose cyclophosphamide and fludarabine regimen that has considerable side effects. The single cyclophosphamide dose mediated mild lymphopenia and moderate neutropenia but did not cause complete lymphodepletion in the patient. In order to protect the infused TIL from toxic effects, we administered cyclophosphamide 4 days before TIL-2 and TIL-3 transfer.

We decided to administer repeated transplants with three escalating doses of relatively low numbers of TIL (TIL-1: 1,4 × 10⁹. TIL-2: 2,0 × 10⁹; TIL-3: 8 × 10⁹ T- cells), instead of a single high-dosed transplant of several-fold 10¹⁰ T-cells. Our treatment modification using repeated transfer of TIL preparations in contrast to single infusions might improve the clinical outcome by prolonging TIL-tumor interactions *in vivo.*

We observed PSA and radiological response after the first and second TIL treatment lasting for 5 and 7 weeks, respectively, indicating that TIL treatment was of transient efficacy. This partial and not durable response might be explained by the low number of TIL, by an impaired persistence of transferred T-cells or by inadequate migration potential of the transferred T-cells into the tumor microenvironment. To improve the outcome, a significantly higher cell number (4-fold to TIL-2) was infused in TIL-3. In addition, we combined TIL-3 treatment with peri-interventional pembrolizumab. In contrast to the first and second TIL treatment, PSA values completely decreased down to < 0,01 µg/L and remained negative up to now 3 years. Closely correlated with the decrease of PSA, the high pre-TIL NY-ESO-1 serum antibody titer continuously decreased over the course of TIL treatment in parallel with disease regression.

We were able to identify specificity for NY-ESO-1 in each of the three TIL transplants. Additional TIL specificities for peptides derived from individual tumor mutational proteins PRPF8, TRPS1 and the androgen receptor splice variant-12 were identified in all three TIL preparations. AR-V12 induces a ligand independent expression of prostate specific antigens (KLK-3, KLK-2, FOLH1) whose overexpression was observed at mRNA level. The corresponding proteins represent potential immune targets, their targeting may have contributed to the durable clinical response to treatment in this patient. The peptide panel used for the identification of TIL specificities did not represent the full range of TAA and neo-antigens expressed in the tumor. T-cells reactive against other target antigens, e.g., MAGE that was also expressed in the tumor, may have contributed to the clinical outcome, too. Nevertheless, it seems unlikely that quantity, quality and specificity of TIL-3 have exclusively mediated to the durable outcome. It is also unlikely that tumor response and clinical improvement is solely attributable to immune-checkpoint blockade with pembrolizumab for the following reasons. First, we observed clinical and radiological response after TIL-1 and TIL-2 treatment, without pembrolizumab. Second, patient's tumor was classified to exhibit MSS (microsatellite stability) and a low TMB, and third, PD-L1 was low thus making response to immune-checkpoint blockade alone unlikely.

In the present study we demonstrate that prostate cancer-derived TILs can be successfully isolated and effectively expanded *in vitro.* TILs were reactive against NY-ESO-1 and several individual tumor antigens. Complete and durable tumor response may has resulted from the combination of TILs treatment with immune-checkpoint blockade that might have helped to overcome PD-1/PD-L1 mediated T-cell inhibition in the tumor microenvironment. With respect to different immune escape mechanisms, TILs treatment targeting several tumor antigens may be advantageous over monospecific immunotherapeutic strategies like cancer vaccines or CAR-T-cell treatment.

In conclusion, adoptive transfer of TILs represents a promising immunotherapeutic approach also for tumors with low TMB like mHRPC. Further investigation in larger patient populations in clinical studies, including precise analysis of tumor antigen - and T-cell repertoire, are warranted.

### General

It should be understood that any feature and/or aspect discussed above in connections with the compounds according to the invention apply by analogy to the methods described herein.

The following figures and examples are provided below to illustrate the present invention. They are intended to be illustrative and are not to be construed as limiting in any way.

### BRIEF DESCRIPTION OF THE FIGURES

**Figure 1****. TIL treatment and correlation with immunological and clinical responses. a: Treatment schedule.** Time point of each TIL administration dose was defined as day 0. The patient received conditioning reparative non-myeloablative lymphodepleting chemotherapy (CTX) with cyclophosphamide (60 mg/kg) on day -1 (TIL-1) and (30 mg/kg) on day -4 (TIL-2 and TIL-3) before intravenous (i.v.) infusion of the TIL supported by administration of IL-2 (aldesleukin) at 600.000 IU/kg. IL-2 was infused 5 times every 12 hours starting sixteen hours after each TIL infusions. Four doses of pembrolizumab (1 mg/kg) were administered within the third TIL treatment (TIL-3) on days - 7, + 15, + 59 and + 80. TIL were expanded with IL-2, IL-15 and IL-21 for the first and second TIL infusions while just IL-2 was used for the expansion of the third TIL infusion. **b: PSA values:** Shortly after the first TIL treatment, PSA decreased from 33 to 18 µg/l within a period of 5 weeks, then rose quickly up to 34. After the second TIL treatment PSA decreased for a period of 7 weeks down to 5 µg/l, then rose again up to a maximum level of 35 µg/l. After the third TIL treatment PSA level continuously decreased within 8 weeks to < 0,01 µg/l and has not been detectable since. **c: NY-ESO-1 serum antibody.** The patient showed a high pre-TIL treatment antibody. During and after the course of TIL treatment the antibody continuously decreased and is currently detectable only on residual level. OD values are shown for 1:400 serum dilution. ( ) cut off OD in healthy donor serum. **d: Imaging.** MRI/CT images were obtained 3 months and 1 day before TIL-1 treatment (pre-TIL), 2 weeks and 6 weeks after TIL-1 and TIL-2, respectively and one day before TIL-3 treatment (pre-TIL-3). The tumor significantly regressed 8 weeks after TIL-3. PET-CT/CT show that the tumor mass completely resolved 1 year after TIL-3 (PET/CT). The patient continued to demonstrate complete response 3 years after TIL-3 treatment up to now.

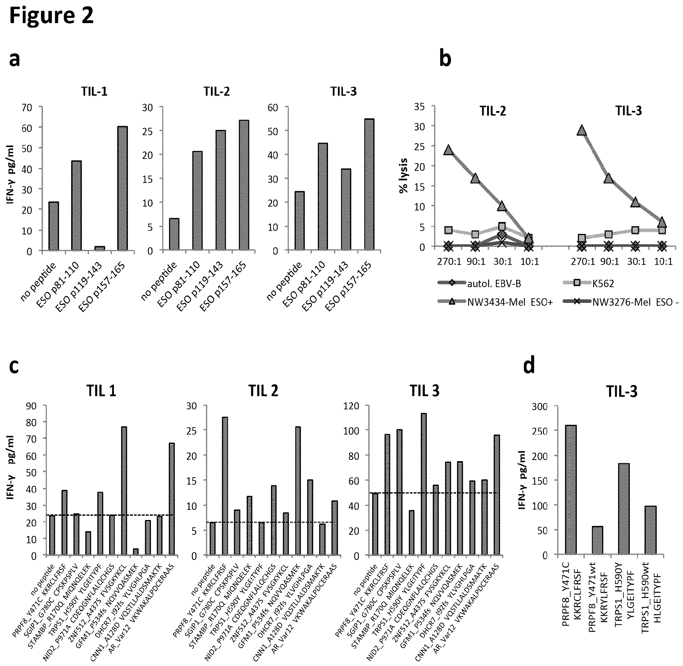

**Figure 2****: TIL specificity.** IFN-γ production of TIL was measured in ELISA. a: specific reactivity for NY-ESO-1 detectable in all three TIL infusions, in particular for the HLA-A2 restricted epitope NY-ESO-1 p157-165, for NY-ESO-1 20mer peptides p81-100 and p91-110, and for NY-ESO-1 24mer peptide p119-143 **(****Figure 2a****)**. TIL showed specific cytotoxicity against NY-ESO-1/HLA-A2 expressing tumor cells in standard chromium-51 (Cr⁵¹) release assay **(****Figure 2b**). Reactivity was also demonstrated for several mutational peptides: KKRCLFRSF (SEQ ID NO: 12) derived from gene PRPF8 (TIL-1, TIL-2, TIL-3), YLGEITYPF (SEQ ID NO: 15) from gene TRPS1 (TIL-1, TIL-3) FVSGKYKCL (SEQ ID NO: 17) derived from gene ZNF512 (TIL 1,3), NQWQASMEK (SEQ ID NO: 18) from gene GFM1 (TIL 2,3), CPSKPSPLV (SEQ ID NO: 13) from gene SGIP1 (TIL 3) and peptide VKWAKALPDCERAAS (SEQ ID NO: 21) derived from a part of the unique C-terminus of the V12 splice variant AR-V12 (TIL 1,2,3). Given that TIL-1 and TIL-2 infusions were generated from the same pre-expansion culture, these results show that the immunological phenotype of TIL comprised of NY-ESO-1 specific T-cells in all three TIL infusions and of 4 and 6 mutation specific T-cells in TIL1/2 and TIL3, respectively **(****Figure 2c****).** T-cells of TIL-3 infusion were also analyzed for recognition of the wild type peptides derived from genes PRPF8 and TRPS1, respectively. IFN-γ production was significantly higher for the mutant versus the wild-type peptide **(****Figure 2d****).**

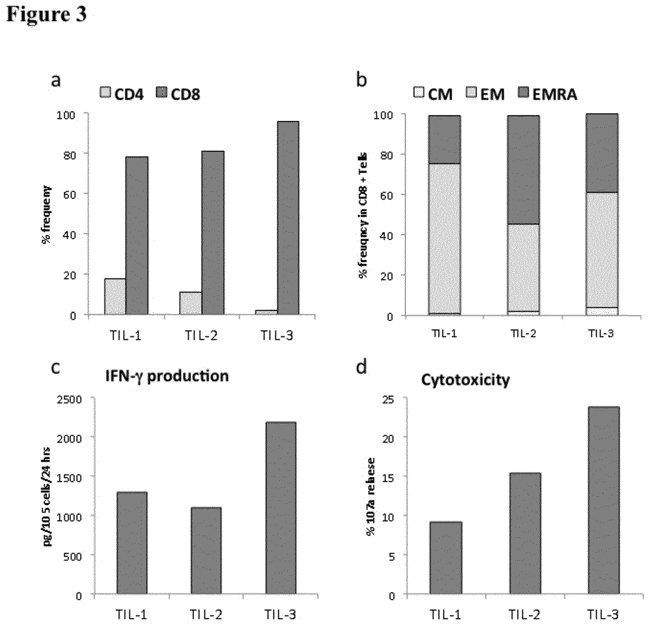

**Figure 3****: TIL phenotype and function.** TILs were mostly CD8 (a) and the majority of CD8 were effector memory (EM) T-cells (b). IFN-gamma release of TILs after 24-hours stimulation with OKT3 (c). PMA induced CD107a expression in TILs showing cytotoxic potential (d). CM= central memory T-cells, EMRA = terminal effector memory T-cells.

### MATERIALS AND METHODS

### TIL production: isolation, cultivation, and expansion

Surgical tumor biopsies were taken in June 2018 (TIL-1 and TIL-2 starting material) and in January 2019 (TIL-3). Tissue samples were sliced into pieces of around 1 mm3 and placed in a GMP-compliant and completely closed perfusion bioreactor system, operated on a platform of GMP Bredder, Control Unit and belonging software. TIL expansion started in a 30MM perfusion bioreactor (phase 1) and continued in a 500MM perfusion bioreactor (phase 2). The culturing process is automatically steered by an algorithm based on pH-, hyperoxic pO2 and temperature providing fresh media to the upgrowing cells. Cellgenix medium with 10% AB human serum and provides up-growing TIL were isolated from the tumor specimen and cultured in medium containing IL-2 (1000 IU/mL), IL-15 (180 IU/mL) and IL-21 (1 IU/mL) (Miltenyi, Bergisch Gladbach, Germany) first in 24-well plates in Cellgro medium supplemented with human serum (10%), anti-human CD3 antibody (clone OKT3, Miltenyi) and allogeneic, 55-Gy irradiated feeder cells added on day 3 (1×10⁶ cells), followed by rapid expansion using OKT3 (30µg/mL) and allogeneic, 55Gy-irradiated feeder cells. GMP-scale production of TIL for clinical use was carried out by Zellwerk GmbH (Berlin, Germany) using the ISO 13485-certified close perfusion bioreactor cell cultivation platform for advanced therapeutic medicinal products (ATMPs). Culture medium for TIL-1 and TIL-2 expansion was supplemented with IL-2, IL-15 and IL-21 (Miltenyi, Bergisch Gladbach, Germany), while TIL-3 expansion was carried out in IL-2 medium only. In a short initiation phase, anti-human CD3 antibody (clone OKT3, Miltenyi) and a low amount of allogenic feeder cell, irradiated with 55-Gy, were added once. Outgrowth of TIL from tumor slices takes place under controlled circulation of the medium in the bioreactor vessel and maintenance of a proven ratio of circulating to freshly supplied medium. Numbers of cells were routinely counted, and glucose and lactate concentrations estimated. At around a density of 1-3 × 10⁹ TILs in a 30MM bioreactor, TILs were separated from tissue material by transferring the by gravity over the mesh port into a single use 500M perfusion bioreactor. During this phase, automatic feeding with medium supplemented with 10% AB human serum and interleukins was continued until harvest of the TIL. Thus, no additional stimulus was made causing changes in the phenotypes formed during phase 1. Cells were washed twice with 0,9 % saline solution, resuspended in 5 %albumin solution, and transferred into infusion bags. Bags were transported to the patient in a biospecimen handling unit at ambient temperature 15°-22°C. Back-up TIL samples, if any, were suspended in 90% human AB serum with 10% dimethyl sulfoxide (DMSO) for storage in liquid nitrogen.

### Flow cytometry and functional characterization of TIL

TIL phenotype, Treg-cells and CD107a induction was determined using Beckman Coulter DURAClone IM T-cell subset and DuraClone IM Treg tube kits. CD107a induction was performed using Beckman Coulter DURActive 1 for cellular activation and stained with anti-human CD107a PE antibody (clone H4A3), anti-human CD3 PE-Cy7, anti-human CD4 V450 and anti-human CD8α APC-Cy7 antibodies. Acquisition of events was performed in a CytoFlex flow cytometer from Beckman Coulter. IFN-γ production was measured after stimulation of TIL with OKT3 for 24 hours followed by cytokine quantification of the culture supernatant by enzyme-linked immunosorbent assay (ELISA).

### Immuno-histochemistry (IHC)

Tumor tissue sections were cut, deparaffinized and treated with 3% H₂O₂ in order to block endogenous peroxidase activity and to 'demask' for antigen retrieval. The primary monoclonal mouse anti-NY-ESO-1 (clone E978) provided by the Monoclonal Antibody Core Facility (MACF) at Sloan Kettering Institute for Cancer Research, diluted in antibody diluent, was added for 30 min. Sections were then incubated with horseradish peroxides-conjugated ENVision for 30 min. The final reaction was visualized by incubation with diaminobenzidine + substrate-chromogen, followed by counterstaining of sections with hematoxylin²⁶. Tissue from testis served as positive control.

### IFN-γELISpot

PBMCs were isolated by Ficoll/Hypaque density-gradient centrifugation (GE Healthcare) and cryopreserved with 10% DMSO in FBS until time of analysis. PBMCs were pre-stimulated *in vitro* in 48-well-plates at 1,2 × 10⁶cells/well with NY-ESO-1, with neo-antigen short and long peptides and with CEF-MHC class I control peptide pool (PANATecs). After one round of re-stimulation on day 7, the pre-sensitized T-cells were analyzed between days 10 and 12 in IFN-γ ELISpot for specific recognition of the peptides pulsed on autologous or HLA-matched allogeneic antigen-presenting cells (APC) e.g., autologous DCs and/or EBV-transformed B cells, T2. A response was considered positive if the number of spots in the peptide-exposed well was two-fold higher or more than the number of spots in the non-stimulated control well, with a minimum of ten peptide-specific spots/25,000 T-cells after subtraction of background spots.

### IFN-γ ELISA

TIL or PBMC were incubated with peptides (1µg/ml) in round-bottom 96-well microtiter plates in 200 µl TCM for 3 - 7 days at 37°C with 5% CO₂. Culture supernatants (100µl/well) were harvested and analyzed for IFN-γ production in flat bottom 96-well multi plates using the IFN-γ ELISA kit from Mabtech AB (#3420-1A-6) according to the manufacturer's instructions.

### Nucleic acid isolation, whole-exome sequencing and neoepitope selection

Genomic DNA from tumor tissue and corresponding normal blood was purified by DNeasy Blood & Tissue Kit (Qiagen, Cat #: 69504). All DNA isolations included the optional RNAse-A digestion step. DNA exome sequences were enriched from tumor and normal DNA using the Ion Torrent AmpliSeq^{™} Exome RDY Kit (Thermo Fisher Scientific, Carlsbad, CA, Cat #: A38264) and the Ion AmpliSeq^{™} Library Preparation Kit plus (Thermo Fisher Scientific, Cat #: 4488990). Barcoded libraries were quantified by Ion Library TaqMan^{™} Quantification Kit (Thermo Fisher Scientific, Cat #: 4468802). Tumor- and normal DNA-derived libraries were adjusted at a molecular ratio of 2:1. Sequencing was done using Torrent Suite^{™} Software 5.6 and 200 bp OT2 kit on Ion GeneStudio^{™} S5 System using a Chip 540 delivering 80-90 million reads per exome pair. The reads were aligned to the human genome reference sequence (hg19) using integrated torrent suite algorithm. The typical average coverage was 120x in the tumor versus 60x in normal DNA. Output BAM files were screened to identify point mutations, small insertions, deletions and stop codons by Ion Reporter Software using AmpliSeq Exome tumor-normal pair workflow with filter focus on Ion AmpliSeqExome HiQ region. Minimal requirements for mutations to be included in further analysis were: coverage of more than 49x in tumor tissue and more than 19x in normal DNA with a frequency of mutation >10% in the tumor with no single affected read in the accompanying normal DNA sample. Corresponding protein sequences were translated by an algorithm developed in-house for SNPs which allows constructing 31-mer wildtype and mutated peptides, respectively by placing the mutation at the center of the sequence. Indels were separately translated, by using the "codong_change.pl" function in the ANNOVAR software, which was also used to annotate mutations and to exclude variances with significant presence in the human genome database (kaviar_20150923, with genomic variances in 64K exomes). For the evaluation of expressed genes, total RNA was extracted from tumor slices using the RNeasy FFPE Kit (Qiagen, Cat #: 73504), transcribed, amplified and cyanine-3-stained according to the Gene Expression FFPE Workflow Guide (Agilent Technologies, Cat #: G4112-90000). Stained cDNA was hybridized to Human GE 4x44 v2 Microarray slides (Agilent, Cat #: G4845) and scanned on a DNA Microarray Scanner (Agilent, Cat #: G2505C). Mutation-bearing peptides corresponding to higher mRNA expressions were favored for downstream selection. HLA typing (HLA A/B/C and HLA-DRB1 loci at least at 4 -digit resolution) was done using EDTA blood at the Institute for Medical Diagnostics (IMD), Berlin-Potsdam, Germany. 9-mer peptides carrying putative HLA class-I neoepitopes were selected by a prediction score from the netMHC-4.0 and IEDB MHC-I--2.17 software packages. For the HLA class II locus, 15 to 17-mer peptides were selected using the netMHCIIpan-3.1 and IEDB MHCII-2.17.3) (using rank score of the method "IEDB recommended" in IEDB algorithm resp.) programs. Peptides carrying mutations in the predicted core region of HLA class II are preferred. Peptides were synthesized and HPLC-purified by Intavis Bioanalytical Instruments and Peptide Services (Cologne, Germany) with a purity threshold of at least 90% (mostly > 98%) using MALDI-MS and RP-HPLC (214 nm).

### Digital PCR for detection of residual disease

For high specific detection of residual disease, we analysed mutation frequency in circulating free DNA. We selected a single nucleotide variant (SNV) in RFPL1 Gene (RFPL1, chr22:29835153 G>T, NM_021026, p.Val125Leu, c.G373T) for deep mutation analysis, which was found to be one of the most predominant SNPs in patients tumor DNA by whole exome sequencing, referring on a frequency of 64%. The SNV was proven to be somatic by comparison with patients PBL DNA. Primers (SEQ IS NOs: 1 and 2) and dual labelled LNA probes (SEQ ID NO: 3 and 4) were designed with OligoArchitect^{®} Online (Sigma-Aldrich) and synthesized by Integrated DNA Technologies Belgium. Cf DNA was extracted from 20ml blood taken into cfDNA BCT^{®} CE tubes (STRECK La Vista, NE USA) and QIAamp^{®} Circulating Nucleic Acid KIT (QIAGEN, Hilden Germany). Digital PCR was performed on a QIAquite Cube system using a 26k nanoplate. Amplification parameters were (2 min 95°C, 40x (15 sec 95°C, 15 sec 58°C 30 sec 72°C).

### EXAMPLES

### Example 1 - Clinical study

### Case

A now 72-year-old man was first diagnosed with prostate cancer in 12/2004. He underwent complete prostatic vesiculectomy and pelvic lymphadenectomy. The disease was classified as pT2c, N0 Mx G3 R1, Gleason-Score 9 (= 4 + 5). Radiotherapy was administered postoperatively to bone metastasis with a total dose of 67 Gy. He received various antihormonal treatments until the disease progressed in 5/2018 infiltrating urethra, penis root and lymph nodes. At that time, the patient refused standard chemotherapy and was therefore evaluated for experimental TIL treatment on the basis of a single patient compassionate use treatment. As a debulking approach and to isolate TIL, the soft tissue tumor metastasis was sub-totally resected in 6/2018. NY-ESO-1 expression of the tumor was confirmed by RT-PCR and immunohistochemistry. Twelve weeks later, a first TIL infusion (TIL-1) was administered consisting of 1400 Mill T cells followed by a second TIL infusion (TIL-2) of 2000 Mill T cells two months later. The tumor was re-biopsied in 1/2019 for generation of a new batch of TIL. NY-ESO-1 expression was also confirmed on that biopsy. A third TIL infusion (TIL-3) was administered in 3/2019 at a dose of 8000 Mill T cells. Prior to each TIL infusion the patient received conditioning chemotherapy with cyclophosphamide on d-1 at 60 mg/kg (TIL-1) and on d-4 at 30 mg/kg (TIL-2, TIL-3). TIL infusions were supported by interleukin (IL-2), given 5 times every 12 hours at 600,000 U/kg, starting 12 hours after TIL infusion. One week before TIL-3 infusion, checkpoint blockade with Pembrolizumab (1 mg/kg) was initiated for a total of 4 doses given every 3 weeks. An overview of the treatment schedule is provided in Figure 1a. PSA levels decreased after each of the three TIL infusions. A complete remission was first recognized by the decrease of PSA down to 0.01 µg/ml two months after TIL-3 infusion (Figure 1b) in parallel with the development of a complete remission of all tumor manifestations. Concomitantly the high pre-TIL NY-ESO-1 serum antibody titer continuously decreased over the course of TIL treatment (Figure 1c). MRT images show that the tumor burden had reduced significantly 8 weeks after administration of TIL-3. The patient's most recent imaging shows complete and sustained tumor remission lasting for now 3 years (Figure 1d). Corresponding to the clinical picture of complete remission a mutation variant RFPL1 gene of the tumor was not detectable in the patient's current plasma by using circulating free DNA (cfDNA) digital PCR analysis.

### NY-ESO-1 immunity

NY-ESO-1 expression of the tumor was confirmed by RT-PCR and immunohistochemistry, showing strong positivity in both tumor biopsies used for TIL isolation. Serum samples were collected before the first TIL treatment and continuously over the entire treatment period until today. Longitudinal measurement of NY-ESO-1 antibody was performed by standard Elisa. The first serum available from the year 2006 was negative for NY-ESO-1 antibodies. Three months before TIL treatment, the patient presented with a strong NY-ESO-1 antibody titer along with significant disease progression. During and after the course of TIL treatment the antibody continuously decreased in parallel with decreasing tumor burden and is currently detectable on residual titer level only **(****Figure 2a****).** PSA values were routinely measured over the course of disease to monitor clinical development and response to treatment. Shortly after the first TIL treatment, PSA decreased from 33 to 18 µg/l within a period of 5 weeks, then rose quickly up to 34. After the second TIL treatment PSA decreased for a period of 7 weeks down to 5 µg/l, then rose again up to a maximum level of 35 µg/l. After the third TIL treatment PSA level continuously decreased within 8 weeks to < 0.01 µg/l and has not been detectable since **(****Figure 2b****).** Partial tumor regression was demonstrated after the first and second TIL treatment; complete tumor regression was achieved 2 months after the third TIL treatment, which has been confirmed now for 18 months **(****Figure 2c****).**

### Phenotype and functional activity of the TIL

Flow cytometric phenotype analysis revealed approximately 78%, 81% and 96% CD8+ T cells in the first (TIL-1) second (TIL-2) and third (TIL-3) infusion, respectively. CD4+ T cells decreased from 18% in TIL-1 to 11% in TIL-2 and 1,7 % in TIL-3 (Figure 3a). TIL were further characterized for expression of costimulatory molecules (CD25/CD28) and differentiation markers (CD45RA, CCR7). The majority of the TIL were effector memory T cells, with 24, 54 and 39% terminal effector memory (EMRA) and < 10% central memory T cells (Figure 3b). Functional activity was evaluated after a 24-hour stimulation of 1 × 10⁵ T cells with OKT3. IFN-γ release of the TIL reached approximately 1298 pg in TIL-1, 1095 pg in TIL-2 and 2184 pg in TIL-3, respectively (Figure 3c). Cytotoxic potential was confirmed by expression of CD107a known to be upregulated after activation. 9.1% of the TIL-1, 15,4 % of TIL-2 and 23.8% of TIL-3, mostly CD8+ T cells, were CD107a+, exhibiting cytotoxic potential (Figure 3d). CD25hi CD127- FoxP3+ regulatory CD4+ T cells (Tregs) were < 0.13% in all TIL preparations.

### Specificity of TIL

Based on the antigenic profile and the strong expression of NY-ESO-1 in the patient's tumor we used a panel of immunogenic 'hot spot' NY-ESO-1 peptides known to be recognized by T-cells of patients with NY-ESO-1 expressing tumors. Besides we used a panel of 10 individual HLA matched peptides derived from mutational antigens identified by WES in the patient's tumor **(Table 1).** This peptide panel represents a selection of 9 peptides of 77 non-synonymous coding neo-antigens detected in the tumor by exome sequencing and the frameshift androgen receptor (AR) alternative splice variant AR-V12, found by Oncomine RNA sequencing. TIL were incubated with these peptides and cell culture supernatant was analyzed for IFN-γ in ELISA assay. Specific reactivity for NY-ESO-1 was found in all three TIL infusions, in particular for the HLA-A2 restricted peptide NY-ESO-1 p157-165, for NY-ESO-1 20mer peptides p81-100 and p91-110 that include 9mer peptides p92-100 and p96-104, known to be presented on HLA-Cw3 and for NY-ESO-1 24mer peptide p119-143 **(****Figure 2a****).** In addition, TIL showed specific cytotoxicity against NY-ESO-1/HLA-A2 expressing tumor cells in standard chromium-51 (Cr⁵¹) release assay **(****Figure 2b**). Reactivity was also demonstrated for several mutational peptides: KKRCLFRSF (SEQ ID NO: 12) derived from gene PRPF8 (TIL-1, TIL-2, TIL-3), YLGEITYPF (SEQ ID NO: 15) from gene TRPS1 (TIL-1, TIL-3) FVSGKYKCL (SEQ ID NO: 17) derived from gene ZNF512 (TIL 1,3), NQVVQASMEK (SEQ ID NO: 18) from gene GFM1 (TIL-2, TIL-3), CPSKPSPLV (SEQ ID NO: 13) from gene SGIP1 (TIL-3) and peptide VKWAKALPDCERAAS (SEQ ID NO: 21) derived from a part of the unique C-terminus of the V12 splice variant AR-V12 (TIL-1, TIL-2, TIL-3) **(****Figure 2c****).** Given that TIL-1 and TIL-2 infusions were generated from the same pre-expansion culture and TIL-3 from a separate one, these results show that the immunological phenotype of TIL comprised of NY-ESO-1 specific T-cells in all three TIL infusions and of 4 and 6 mutation-specific T-cells in TIL-1, TIL-2 and TIL-3, respectively. T-cells of TIL-3 infusion were also analyzed for specific recognition of the wild type peptides derived from genes PRPF8 and TRPS1, respectively. IFN-γ production was significantly higher for the mutant versus the wild-type peptide **(****Figure 2d****).**

## Claims

1. A dosage regimen for use in the treatment of cancer comprising administration of *in vitro* expanded tumor infiltrating lymphocytes, TILs, to a mammal suffering from cancer at day 0(TIL), followed by administration of a checkpoint inhibitor at day 0(CI), wherein day 0(CI) is in a range of from 1 months to 5 months later than day 0(TIL) such as from 6 weeks to 4 months, from 8 weeks to 3 months or about 2 months later than day 0(TIL).

2. A dosage regimen for use according to claim 1 comprising administration of *in vitro* expanded tumor infiltrating lymphocytes, TIsL, (TIL-2) to a mammal suffering from cancer at day 0(TIL-2), followed by administration of a checkpoint inhibitor at day 0(CI), wherein day 0(CI) is in a range of from 1 months to 5 months later than day 0(TIL-2) such as from 6 weeks to 4 months, from 8 weeks to 3 months or about 2 months later than day 0(TIL-2).

3. A dosage regimen for use according to claim 1 or 2 further comprising administration of *in vitro* expanded tumor infiltrating lymphocytes, TIL, (TIL-3) to said mammal at day 0(TIL-3), wherein day 0(TIL-3) is in a range of from 1 to 14 days after day 0(CI) such as from 2 to 12 days, from 5 to 12 days or 7 days after day 0(CI).

4. A dosage regimen for use according to any one of the preceding claims further comprising administration of *in vitro* expanded tumor infiltrating lymphocytes, TIL, (TIL-1) to said mammal at day 0(TIL-1), wherein day 0(TIL-1) is in a range of from 1 to 4 months before day 0(TIL-2) or day 0(TIL) such as from 6 weeks to 4 months, from 8 weeks to 3 months or about 2 months before day 0(TIL-2).

5. A dosage regimen for use according to any one of the preceding claims, further comprising administration to said mammal of a chemotherapeutic agent from 7 to 1 day before administration of TIL-1, TIL-2 and/or TIL-3.

6. A dosage regimen for use according to claim 5, wherein said chemotherapeutic agent is an agent normally used in the treatment of said cancer, but where the dose is lowered compared to the dose normally used in the treatment of said cancer.

7. A dosage regimen for use according to any one of the preceding claims, wherein the number of TILs increases from TIL-1 to TIL-2 and to TIL-3.

8. A dosage regimen for use according to any one of the preceding claims, where the mammal suffering from cancer has not been subject to chemotherapy in a time period of at least 6 months before start of said treatment of cancer.

9. A dosage regimen according to any one of claims 3-8, wherein said day 0(TIL-3) is determined by monitoring the plasma level of a marker specific for said cancer, a tumor-associated antigen specific for said cancer or a neoantigen specific for said cancer, wherein the time period for monitoring starts after administration of TIL-2, and when the level increased by a factor 4 or more, day 0(TIL-3) is 1-7 days after such an increase has been observed.

10. A dosage regimen according to any one of claims 4-9, wherein said day 0(TIL-2) is determined by monitoring the plasma level of a marker specific for said cancer, a tumor-associated antigen specific for said cancer or a neoantigen specific for said cancer in said mammal, wherein the time period for monitoring starts after administration of TIL-1, and when the level increased by a factor 4 or more, day 0(TIL-2) is 1-7 days after such an increase has been observed.

11. A dosage regimen for use according to any one of the preceding claims, wherein the cancer is selected from cancers that not respond effectively to sole treatment with checkpoint inhibitors such as rhabdoid tumors, Ewing sarcomas, thyroid cancers, AML, medulloblastomas, glioblastomas, neuroblastoma, carcinoid tumors, prostate cancer, breast cancer and pancreas cancer.

12. A dosage regimen according to any one of the preceding claims, wherein the cancer is prostate cancer.

13. A dosage regimen according to claim 12, wherein the level of PSA, and/or NY-ESO-1 is monitored.

14. A dosage regimen for use in the treatment of a mammal suffering from cancer, the dosage regimen comprises
i) administration to said mammal *in vitro* expanded tumor infiltrating lymphocytes to the patient at day 0(TIL-1),
ii) administration to said mammal *in vitro* expanded tumor infiltrating lymphocytes to the patient at day 0(TIL-2), wherein day 0(TIL-2) is in a range of from 1 to 4 months after day 0(TIL-1) such as from 6 weeks to 4 months, from 8 weeks to 3 months or about 2 months after day 0(TIL-1),
iii) administration to said mammal a checkpoint inhibitor at day 0(CI), wherein day 0(CI) is in a range of from 1 months to 5 months later than day 0(TIL-2) such as from 6 weeks to 4 months, from 8 weeks to 3 months or about 2 months later than day 0(TIL-2),
iv) administration to said mammal *in vitro* expanded tumor infiltrating lymphocytes, TIL, (TIL-3) to said mammal at day 0(TIL-3), wherein day 0(TIL-3) is in a range of from 1 to 14 days after day 0(CI) such as from 2 to 12 days, from 5 to 12 days or 7 days after day 0(CI).

15. A dosage regimen for use according to any one of the preceding claims, wherein said *in vitro* expanded tumor infiltrating lymphocytes are obtained by providing a tumor or tumor metastasis sample from said mammal and subjecting said sample to *in vitro* culturing.

16. A dosage regimen for use according to any one of the preceding claims, wherein at least 80% of said TIL-1, TIL-2 and/or TIL-3 are CD8 positive and at least 20% of the CD8 positive TILs are effector memory cells.
